(19) Europäisches Patentamt
European Patent Office
Office européen des brevets

(11) Veröffentlichungsnummer: **0 057 827**
**B1**

(12) # EUROPÄISCHE PATENTSCHRIFT

(45) Veröffentlichungstag der Patentschrift: **13.08.86**

(51) Int. Cl.⁴: **C 07 D 213/85**, C 07 D 213/80

(21) Anmeldenummer: **82100302.7**

(22) Anmeldetag: **18.01.82**

(54) **Verfahren zur Herstellung von 2-Aminopyridinderivaten.**

(30) Priorität: **30.01.81 DE 3103065**

(43) Veröffentlichungstag der Anmeldung: **18.08.82 Patentblatt 82/33**

(45) Bekanntmachung des Hinweises auf die Patenterteilung: **13.08.86 Patentblatt 86/33**

(84) Benannte Vertragsstaaten: **BE CH DE FR GB IT LI NL**

(56) Entgegenhaltungen:
**DE-A-1 811 973**
**DE-A-2 365 302**
**DE-A-2 424 373**

**SYNTHESIS Mai 1979, Seiten 376-378, Stuttgart, DE. G. EGE et al.: "Eine neue Synthese von 4-Dimethylamino-1,3-butadien-1,1-dicarbonitrilen und deren Cyclisierung zu 2-Amino-3-cyanopyridinen"**

(73) Patentinhaber: **BASF Aktiengesellschaft, Carl-Bosch- Strasse 38, D-6700 Ludwigshafen (DE)**

(72) Erfinder: **Acker, Rolf- Dieter, Dr., Tuchbleiche 8, D-6906 Leimen (DE)**
Erfinder: **Hamprecht, Gerhard, Dr., Rote- Turm- Strasse 28, D-6940 Weinheim (DE)**

**EP 0 057 827 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Herstellung von 2-Aminopyridinderivaten durch Umsetzung von quaternären Ammoniumverbindungen mit Malodinitril in Gegenwart von Alkanolen, anschließende Umsetzung mit Ammoniak in Gegenwart von Alkanolen, Wasser und/oder Ethern zum Aminoicotinsäurenitril und gewünschtenfalls durch anschließende Umsetzung mit Wasser in Gegenwart von Alkaliverbindungen zur Aminoicotinsäure.

Es ist bekannt, quaternäre Ammoniumverbindungen, die gleichzeitig Derivate des Malonaldehyds sind, der Formel

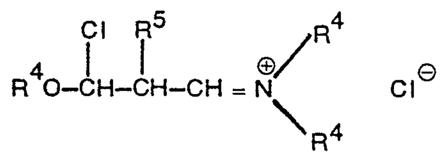

$R^4$ = Alkyl

$R^5$ = Alkyl oder H

durch Umsetzung von Phosgen mit einem Dialkylformamid zum Dialkylformamidchlorid und anschließender Umsetzung mit einem Enolether herzustellen (DE—OS 24 24 373).

Es ist weiterhin bekannt (Synthesis 1979, Seiten 376 bis 378), daß man Alkylidenmalodinitrile mit Lithium-diisopropylamid in Tetrahydrofuran bei −65°C und mit anschließender Umsetzung mit Dimethylformamiddichlorid zu 4-Dimethylamino-1,3-butadien-1,1-dicarbonitrile umsetzt. Diese Butadienverbindungen können durch Reaktion mit Ammoniak in Methanol zu 2-Aminopyridinen umgewandelt werden.

Auf ähnliche Weise wird, wie die DE—OS 18 11 973 beschreibt, aus 2-Methylthio-2-(3,4-dihydro-1-pyrrolidino-2-naphthyl)-ethylen-1,1-dicarbonitril durch Umsetzung mit wäßriger Ammoniaklösung in Gegenwart von Methanol 2-Amino-5,6-dihydro-4-methylthio-benzochinolin-3-carbonitrilerhalten.

Es wurde nun gefunden, daß man 2-Aminopyridinderivate der Formel I

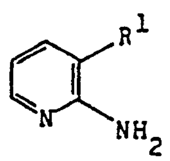

(I)

worin $R^1$ eine Cyanogruppe oder eine Carboxylgruppe bedeutet, vorteilhaft erhält, wenn man quaternäre Ammoniumverbindungen der Formel II

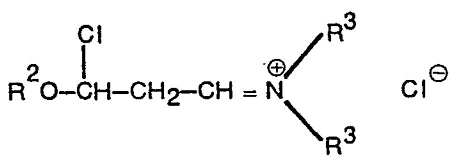

(II)

worin die einzelnen Reste $R^2$ und $R^3$ gleich oder verschiden sein können und jeweils einen aliphatischen Rest bedeuten,

a) mit Malodinitril in Gegenwart von Alkanolen umsetzt und dann

b) das so erhaltene Reaktionsgemisch mit Ammoniak in Gegenwart von Alkanolen, Wasser und/oder Ethern umsetzt und gewünschtenfalls dann

c) das so erhaltene Aminonicotinsäurenitril mit Wasser in Gegenwart von Alkaliverbindungen umsetzt.

Die Umsetzung kann für den Fall der Verwendung von N,N-(3-Chlor-3-ethoxypropyliden)-N,N-dimethyl-ammonium-chlorid durch folgendes Schema wiedergegeben werden:

a)

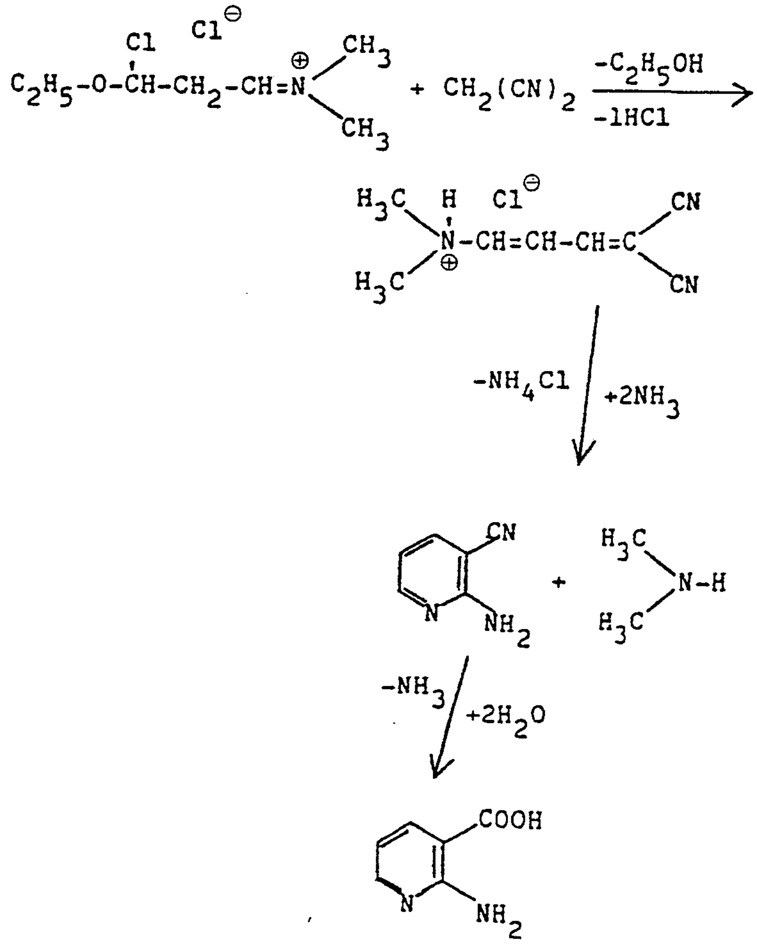


Im Hinblick auf die bekannten Verfahren liefert das Verfahren nach der Erfindung auf einfacherem und wirtschaftlicherem Wege Aminonicotimsäure und sein Nitril in guter Ausbeute und Reinheit und besserer Gesamt-Raum-Zeit-Ausbeute. Schwer zugängliche Reaktionskomponenten, wie Lithiumverbindungen, werden vermieden. Sowohl Nitril wie Säure können einbadig ohne Isolierung der Zwischenstufen hergestellt werden. Alle diese vofteilhaften Ergebnisse sind im Hinblick auf den Stand der Technik überraschend.

Der Ausgangsstoff II ist leicht nach dem in der DE—OS 24 24 373 beschriebenen Verfahren herstellbar. Er kann mit dem Malodinitril in stöchiometrischer Menge oder im Überschuß, zweckmäßig in einem Verhältnis von 0,5 bis 3,0, vorzugsweise 0,5 bis 1,5 Mol Malodinitril je Mol Ausgangsstoff II, umgesetzt werden. Bevorzugte Ausgangsstoffe II und dementsprechend bevorzugte Endstoffe I sind solche, in deren Formeln die einzelnen Reste $R^2$ und $R^3$ gleich oder verschieden sind und jeweils winen Alkylrest mit 1 bis 7, vorzugsweise 1 bis 4 Kohlenstoffatomen, insbesondere Methyl oder Ethyl bedeuten und $R^1$ eine Cyano- oder Carboxylgruppe bezeichnet. Die vorgenannten Reste können noch durch unter den Reaktionsbedingungen inerte Gruppen, z.B. Alkyl- oder Alkoxy-gruppen mit 1 bis 4 Kohlenstoffatomen, substituiert sein.

So sind beispeilsweise als Ausgangsstoff II geeignet: N,N-(3-Chlor-3-äthoxypropyliden)-N,N-dimethyl-ammoniumchlorid; homologe N,N-Diethyl-, N,N-Dipropyl-, N,N-Diisopropyl-, N,N-Dibutyl-, N,N-Diisobutyl-, N,N-Di-sek.-butyl-, N,N-Di-tert.-butyl-derivate; entsprechende 3-Methoxy-, 3-Propoxy-, 3-Isopropoxy-, 3-Butoxy-, 3-Isobutoxy-, 3-sek.-Butoxy-, 3-tert.-Butoxy-derivate.

Die in a) und b) verwendeten Alkanole sind zweckmäßig gleich und können 1—10, insbesondere 1—6 Kohlenstoffatome enthalten. Beispielsweise sind Methyl-, Ethyl-, Propyl-, Isopropyl-, Butyl-, Isobutyl-, sek.-Butyl-, tert.-Butyl-alkohol, bevorzugt Methanol, geeignet. Zweckmäßig verwendet man von 1 bis 1 000, insbesondere 10 bis 100 Mol Alkanol je Mol Ausgangsstoff II. Man kann dad gesamte Alkanol in der Stufe a) einsetzen oder jeweils einen Anteil von zweckmäßig 20 bis 80 Gewichtsprozent des Gesamtalkanols in der Stufe a) und den restlichen Anteil in der Stufe b) zugeben. Ammoniak kann gasförming, flüssig oder zweckmäßig in Gestalt seiner 10- bis 30-gewichtsprozentigen wäßrigen Lösung verwendet werden, vorteilhaft in einer Menge von 2 bis 200, insbesondere 5 bis 50 Mol Ammoniak je Mol Ausgangsstoff II.

In Stufe b) können Alkanole, Ether und/oder Wasser verwendet werden. Als Ether kommen z.B. in Frage Ethylpropylether, Methyl-tert.-butylether, n-Butylethylether, Di-n-butylether, Diisobutylether,

Diisoamylether, Diisopropylether, Anisol, Phenetol, Cyclohexylmethylether, Diethylether, Ethylenglykoldimethylether, Tetrahydrofuran, Dioxan, Thionanisol, ß,ß'-Dichlordiethylether; und entsprechende Gemische. Zwechmäßig verwendet man Wasser oder Ether in einer Menge von 400 bis 10 000 Gewichtsprozent, vorzugsweise von 500 bis 1 000 Gewichtsprozent, bezogen auf Ausgangsstoff II.

Gewünschtenfalls kann in der Stufe c) die Aminonicotinsäure hergestellt werden. Man verwendet als Alkaliverbindung zweckmäßig Alkalihydroxide, Alkalicarbonate, Alkalialkoholate, insbesondere die Natrium- und Kaliumverbindungen. Es sind beispielsweise geeignet: Kaliumhydroxid, Natriumhydroxid, Kaliumcarbonat, Natriumcarbonat, Lithiumhydroxid, Lithiumcarbonat, Natriumbicarbonat, Kaliumbicarbonat, Natriumformiat, Natriumacetat, Natriumpropionat, Natriumbutyrat, Natriumisobutyrat, Kaliumformiat, Kaliumacetat, Kaliumpropionat, Kaliumbutyrat, Kaliumisobutyrat, Natriummethylat, Natriumethylat, Natriumpropylat, Natriumisopropylat, Natriumbutylat, Natriumisobutylat, Natrium-sek.-butylat, Natrium-tert.-butylat, Natriumäthylenglykolat, Natriumpropylen-(1,2)-glykolat, Natriumpropylen-(1,3)-glykolat, Natriumdiäthylenglykolat, Natriumtriäthylenglykolat, Natriumdipropylen-(1,2)-glykolat, Kaliummethylat, Kaliumethylat, Kalium-n-propylat, Kaliumisopropylat, Kalium-n-butylat, Kalium-isobutylat, Kalium-sek.-butylat, Kalium-tert.-butylat, Kaliumäthylenglykolat, Kaliumpropylen-(1,2)-glykolat, Kaliumdiäthylenglykolat, Kaliumtriäthylenglykolat, Kaliumdipropylen-(1,2)-glykolat. Die Umsetzung wird zweckmäßfig mit einer Menge von 0,5 bis 5, vorzugsweise von 0,8 bis 1,5 Äquivalenten alkalischer Verbindung, bezogen auf ein Mol Ausgangsstoff II, durchgeführt. Im allgemeinen verwendet man in Stufe c) Wasser als Lösungsmittel, zweckmäßig 50 bis 500, insbesondere 100 bis 300 Gewichtsprozent Wasser, bezogen auf die Gewichtsmenge Ausgangsstoff II.

Die Umsetzung wird im allgemeinen in der Stufe a) bei einer Temperatur von 40 bis 140, insbesondere 60 bis 120°C, in der Stufe b) bei einer Temperatur von 50 bis 200, insbesondere 80 bis 180°C, in der Stufe c) bei einer Temperatur von 50 bis 150, insbesondere 80 bis 120°C, drucklos oder unter Druck, kontinuierlich oder diskontinuierlich durchgeführt. Zusätzliche organische Lösungsmittel werden im allgemeinen nich benötigt.

Die Reaktion kann wie folgt durchgeführt werden: Ein Gemisch von Ausgangsstoff II, Malodinitril und Alkanol wird während 2 bis 10 Stunden bei der Reaktionstemperatur der Stufe a) gehalten. Dann wird Ammonial und gegebenenfalls weiteres Alkanol zugesetzt und das Gemisch bei der Reaktionstemperatur der Stufe b) während 0,5 bis 5 Stunden gehalten. Gewünschtenfalls wird dann noch Alkaliverbindung und Wasser zugesetzt, gegebenenfalls nach dem Abdestillieren des Alkanols, und das Gemisch während 2 bis 8 Stunden bei der Reaktionstemperatur der Stufe c) gehalten. Der Endstoff kann in üblicher Weise in der Stufe b), z.B. durch Zugabe von organischen Lösungsmitteln und Filtration des Niederschlags, oder nach Stufe c), z.B. durch Ansäuern und Filtration, abgetrennt werden.

Die nach dem Verfahren der Erfindung herstellbaren 2-Aminopyridinderivate sind wertvolle Ausgangsstoffe für die Herstellung von Schädlingsbekämpfungsmitteln, Pharmazeutika, Vitaminen und Farbstoffen. Bezüglich der Verwendung wird auf die vorgenannte Literatur und Ullmanns Encyklopädie der technischen Chemie, Band 8, Seite 503; Band 14, Seite 480; Band 18, Seiten 195—198, verwiesen. Die Endstoffe I sind Ausgangsstoffe für die in der deutschen Offenlegungsschrift 24 30 353 beschriebenen Schädlingsbekämpfungsmittel.

Die in den folgenden Beispielen ganannten Teile bedeuten Gewichtsteile.

### Beispiel 1

a) 100 Teile N,N-(3-Chlor-3-ethoxypropyliden)-N,N-dimethyl-ammoniumchlorid (aus Dimethylformamid, Phosgen und Ethyl-vinyl-ether), 33 Teile Malodinitril und 450 Teile Methanol werden 6 Stunden bei 68°C unter Rückfluß erhitzt.

b) Die Lösung wird abgekühlt und das Lösungsmittel wird im Vakuum abdestilliert. 15 Teile des Rückstands, 80 Teile Methanol und 40 Teile flüssigen Ammoniaks werden in einen Rührautoklaven gegeben und 4 Stunden auf 150°C erhitzt. Nach dem Abkühlen werden Toluol und Ethanol zugefügt, bis ein kristalliner Rückstand verbleibt. Man erhält nach Filtration 6,0 Teile (61% der Theorie) 2-Amino-nicotinonitril vom Fp 129—131°C.

### Beispiel 2

Analog Beispiel 1 wird die Umsetzung mit 114 Teilen N,N-(3-Chlor-3-isobutoxypropyliden)-N,N-dimethyl-ammonium-chlorid (aus Dimethylformamid, Phosgen und Isobutyl-vinyl-ether) durchgeführt. Man erhält nach Filtrations 5,1 Teile (59% der Theorie) 2-Amino-nicotinonitril vom Fp 129 bis 131°C.

### Beispiel 3

Analog Beispiel 1 wird umgesetzt und nach Stufe b) (Erhitzen im Autoklaven) das Lösungsmittel im Vakuum abdestilliert, 20 Teile Wasser und 3 Teile Natriumhydroxid zum Rückstand zugefügt und 5 bis 6 Stunden unter Rückfluß erhitzt. Nach dem Abkühlen und Filtrieren wird mit 10-gewichtsprozentiger Salzsäure angesäuert, abgekühlt und der Niederschlag abgesaugt. Man erhält nach Filtration 5,9 Teile (52% der Theorie) 2-Amino-nicotinsaüre vom Fp 305—310°C.

### Beispiel 4

Analog Beispiel 2 wird umgesetzt und nach Stufe b) (Erhitzen im Autoklaven) das Lösungsmittel

abdestilliert, 15 Teile Wasser und 2,5 Teile Natriumhydroxid werden zum Rückstand zugefügt und 3 bis 4 Stunden unter Rückfluß erhitzt. Nach dem Abkühlen und Filtrieren wird mit 10-gewichtsprozentiger Salzsäure angesäuert, abgekühlt und der Niederschlag abgesaugt. Man erhält nach Filtration 5,0 Teile (49% der Theorie) 2-Amino-nicotinsaüre vom Fp 305—310°C.

Beispiel 5

Analog Beispiel 1 wird nach a) umgesetzt. Nach dem Abkühlen der Lösung werden 120 Teile flüssigen Ammoniaks und 250 Teile Methanol zugegeben und im Rührautoklaven 6 Stunden auf 150°C erhitzt. Nach dem Abkühlen werden Toluol und Ethanol zugefügt bis ein kristalliner Rückstand verbleibt. Man erhält nach Filtration 37 Teile (62% der Theorie) 2-Amino-nicotinsaüre vom Fp 129—131°C.

Beispiel 6

Analog Beispiel 1 wird nach a) umgesetzt. Die Lösung wird abgekühlt und das Lösungsmittel wird im Vakuum abdestilliert. 500 Teile 25-gewichtsprozentige, wäßrige Ammoniaklösung wird zugefügt und im Rührautoklaven 8 Stunden auf 150°C erhitzt. Anschließend werden etwa 380 Teile Lösungsmittel abdestilliert und 20 Teile Natriumhydroxid zugefügt und 4 bis 5 Stunden unter Rüchfluß erhitzt. Nach dem Abkühlen und Filtrieren wird mit 10-gewichtsprozentiger Schwefelsäure angesäuert, abgekühlt und der Neiderschlag abgesaugt. Man erhält nach der Filtration 32 Teile (46% der Theorie) 2-Amino-nicotinsäure vom Fp 305—310°C.

**Patentanspruch**

Verfahren zur Herstellung von 2-Aminopyridinderivaten der Formel I

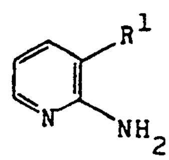

(I)

worin $R^1$ eine Cyanogruppe oder eine Carboxylgruppe bedeutet, dadurch gekennzeichnet, daß man quaternäre Ammoniumverbindungen der Formel II

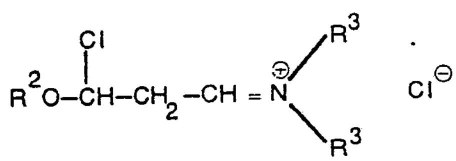

(II)

worin die einzelnen Reste $R^2$ und $R^3$ gleich oder verschieden sein können und jeweils einen aliphatischen Rest bedeuten,

a) mit Malodinitril in Gegenwart von Alkanolen umsetzt und dann

b) das so erhaltene Reaktionsgemisch mit Ammoniak in Gegenwart von Alkanolen, Wasser und/oder Ethern umsetzt und gewünschtenfalls dann

c) das so erhaltene Amino-nicotinsäurenitril mit Wasser in Gegenwart von Alkaliverbindungen umsetzt.

**Revendication**

Procédé pour la préparation de dérivés de 2-aminopyridine de formule I

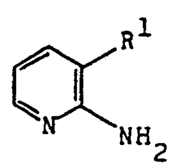

(I)

dans laquelle $R^1$ représente un groupement cyano ou un groupement carboxyle, caractérisé en ce que des composés d'ammonium quaternaires de formule II

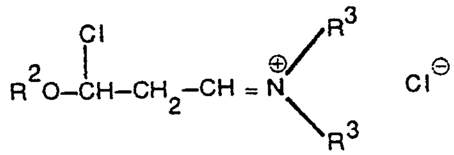

(II)

dans laquelle les différents radicaux $R^2$ et $R^3$ peuvent être semblables ou différents et représentent chacun un radical aliphatique,

a) sont mis en réaction avec le dinitrile malonique en présence d'alcanols, puis

b) le mélange réactionnel ainsi obtenu est mis en réaction avec l'ammoniac en présence d'alcanols, d'eau et/ou d'éthers, puis, au cas où on le désire,

c) le nitrile d'acide aminonicotinique ainsi obtenu est mis en réaction avec l'eau en présence de composés alcalins.

**Claim**

A process for the preparation of 2-aminopyridine derivatives of the formula I

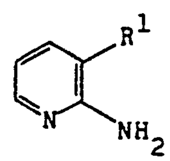

(I)

where $R^1$ is cyano or carboxyl, wherein a quaternary ammonium compound of the formula II

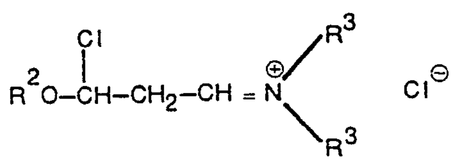

(II)

where the individual radicals $R^2$ and $R^3$ can be identical or different and each is an aliphatic radical,

a) is reacted with malodinitrile in the presence of an alkanol,

b) the reaction mixture thus obtained is then reacted with ammonia in the presence of an alkanol, water and/or an ether and, if desired,

c) the aminonicotinonitrile thus obtained is reacted with water in the presence of an alkali metal compound.